# FASCICULE DE BREVET EUROPEEN

(11) **EP 4 069 817 B1**
(45) Date de publication et mention de la délivrance du brevet: **03.01.2024**
(21) Numéro de dépôt: 20808115.8
(22) Date de dépôt: 19.11.2020
(51) Int. Cl.: C12M 1/107, C12M 1/00

(54) **UNITÉ DE MÉTHANISATION AMÉLIORÉE**
VERBESSERTE METHANISIERUNGSEINHEIT
IMPROVED METHANIZATION UNIT

(30) Priorité: 02.12.2019 FR 1913591
(43) Date de publication de la demande: 12.10.2022
(73) Titulaire: SAXE PARIS INVESTISSEMENT, 75008 Paris (FR)
(72) Inventeur: BORSENBERGER, Eric, 67530 Klingenthal (FR)
(74) Mandataire: Hege, Frédéric
(86) Numéro de dépôt international: PCT/EP2020/082660
(87) Numéro de publication internationale: WO 2021/110419

(56) Documents cités:
- EP-A1- 1 428 868
- WO-A1-89/08616
- WO-A1-2018/091600
- FR-A1- 2 981 086
- US-A1- 2010 159 571

## Description

La présente invention se situe dans le domaine de la production de gaz à partir de matières organiques. Elle concerne plus particulièrement une unité de méthanisation.

La méthanisation est le processus naturel biologique de dégradation de la matière organique en absence d'oxygène. Cette dégradation produit un biogaz, utilisable comme source d'énergie.

La méthanisation par voie sèche discontinue est une méthode dans laquelle on charge une chambre de méthanisation en biomasse. La méthanisation a ensuite lieu. Lorsque la méthanisation est terminée, on décharge la chambre des résidus de biomasse non transformés en biogaz, avant de recommencer. L'usage de plusieurs chambres en parallèle permet une production continue de biogaz.

Afin d'accélérer la méthanisation, il est courant de procéder à une percolation de la biomasse, par exemple en distribuant du lisier sur son dessus. Cet apport de matières chargées en bactéries favorise la dégradation de la biomasse. De plus, étant liquide, le lisier se répand facilement dans toute la biomasse par capillarité et ruissellement.

Lorsqu'une partie du liquide de percolation a complètement traversé la biomasse, un liquide appelé percolat s'accumule dans le bas de la chambre de méthanisation. Ce liquide est composé de la partie du liquide de percolation qui n'est pas restée dans la biomasse, et de certaines particules fines de la biomasse emmenées par ce liquide. L'accumulation de percolat a souvent lieu à l'avant à ou à l'arrière de la biomasse. Il est alors souhaitable de sortir le percolat de la chambre de méthanisation, pour ne pas augmenter la pression sur les parois de la chambre. De plus la sortie du percolat hors de la chambre permet de récupérer un liquide chargé en bactéries qu'on peut réutiliser pour la percolation.

Le document WO2007096392 présente une unité de production de biogaz par voie sèche discontinue. Le sol est incliné afin de drainer le percolat vers une des extrémités de la chambre de méthanisation. A cette extrémité se trouve une grille permettant de récupérer le percolat dans un canal, qui mène à un réservoir de percolat. Cependant le processus de méthanisation n'est pas rapide, et il existe un besoin d'optimisation afin d'accélérer la production de biogaz. EP1428868 divulgue une unité de méthanisation par voie sèche avec un système de percolation comprenant un filtre pour recueillir le percolat sous la biomasse a fermenter.

Un objet de la présente invention est de proposer une unité de méthanisation par voie sèche discontinue permettant une méthanisation plus rapide de la biomasse.

La présente invention a pour objet de répondre au moins en partie aux objets précités en proposant une unité de méthanisation plus efficace, avec un écoulement plus fiable du percolat. A cet effet, elle propose une unité de méthanisation par voie sèche discontinue comportant un espace de chargement destiné à accueillir une biomasse à méthaniser, l'unité de méthanisation comprenant un moyen de percolation, et un moyen d'évacuation d'un percolat, dans laquelle :

- l'unité de méthanisation comporte au moins un premier filtre disposé de sorte à séparer l'espace de chargement d'un espace de circulation,
- l'espace de circulation étant situé entre le premier filtre et le moyen d'évacuation,
- un deuxième filtre étant disposé entre l'espace de circulation et le moyen d'évacuation, ledit deuxième filtre étant configuré plus fin que le premier, de sorte à ne pas laisser passer certains éléments que le premier filtre laisse passer.

Grâce à ces dispositions, le percolat est bien réparti sur la surface du deuxième filtre, dont l'utilisation est ainsi optimisé.

Selon d'autres caractéristiques :
- ledit deuxième filtre peut être disposé de sorte à ménager un deuxième espace de circulation entre ledit deuxième filtre et ledit moyen d'évacuation, permettant ainsi au percolat de traverser ledit deuxième filtre et d'en ressortir librement vers le deuxième espace de circulation, de sorte que l'utilisation dudit deuxième filtre est encore améliré,
- le premier filtre peut ne laisser passer que le liquide et les éléments dont au moins une dimension est inférieure à une dimension grossière, ladite dimension grossière étant comprise entre 10 et 80 mm, ce qui correspond à des dispositions efficaces pour l'invention,
- le deuxième filtre peut ne laisser passer que le liquide et les éléments dont au moins deux dimensions sont inférieures à une dimension fine, la dimension fine étant comprise entre 1 et 10 mm, ce qui correspond à des dispositions efficaces pour l'invention,
- le deuxième filtre peut comporter une cage grillagée dans laquelle sont placés des éléments filtrants tels que des cailloux ou des billes de plastique, ce qui est un mode efficace de réalisation dudit deuxième filtre,
- ladite unité de méthanisation peut comprendre un sol, une paroi avant, une paroi arrière, et deux parois latérales, le moyen d'évacuation étant disposé au niveau de l'intersection entre le sol et la paroi arrière, et le premier filtre s'étendant selon un plan incliné entre le sol et la paroi arrière, d'une paroi latérale à l'autre, ce qui permet un mode de réalisation simple et efficace de l'invention,
- le premier filtre peut comporter des éléments allongés, par exemple en bois, disposés parallèlement entre eux, s'appuyant d'un côté sur le sol et de l'autre côté sur la paroi arrière de l'unité de méthanisation, ce qui représente un mode de réalisation simple et efficace pour retenir la biomasse, et laisser passer le percolat,
- ladite unité de méthanisation peut comporter au moins un moyen d'écoulement couvert disposé au sol le long d'une paroi latérale de l'unité de méthanisation, permettant de faire couler du percolat de la paroi avant vers la paroi arrière sans traverser la biomasse, permettant d'éviter une accumulation de percolat à l'avant de l'unité de méthanisation, qui risquerait d'exercer une pression inacceptable sur la paroi avant.

La présente invention concerne encore un Procédé de percolation d'une unité de méthanisation par voie sèche discontinue comportant successivement les étapes suivantes :
- versement d'un liquide de percolation sur une biomasse, ledit liquide de percolation devenant un percolat en traversant la biomasse,
- filtration du percolat par un premier filtre,
- filtration du percolat par un deuxième filtre plus fin que le premier filtre,
- évacuation du percolat hors de l'unité de méthanisation.

Grâce à ces dispositions, le deuxième filtre est protégé par le premier filtre, et le percolat s'écoule mieux à travers le deuxième filtre.

Selon un mode préféré de réalisation de l'invention, on ajoute en outre une étape de circulation du percolat dans un espace de circulation entre les étapes de filtration du percolat par le premier filtre et par le deuxième filtre ; une telle caractéristique permet d'optimiser le flux du percolat, qui se présente en entrée du deuxième filtre sur toute sa surface, et garantit ainsi une meilleure utilisation dudit deuxième filtre ; de préférence on ajoute encore une étape de circulation du percolat dans un deuxième espace de circulation entre les étapes de filtration du percolat par le deuxième filtre et l'étape d'évacuation du percolat, permettant d'optimiser également le flux de percolat à la sortie du deuxième filtre, et évitant d'obliger le percolat à circuler transversalement dans le deuxième filtre pour arriver jusqu'au moyen d'évacuation.

La présente invention sera mieux comprise à la lecture de la description détaillée qui fait suite, en référence aux figures annexées dans lesquelles :
[Fig.1] La [Fig.1] est une vue en coupe longitudinale d'une unité de méthanisation selon un mode de réalisation préféré de l'invention,
[Fig.2] La [Fig.2] est une vue schématique de l'arrière de l'unité de la [Fig.l],
[Fig.3] La [Fig.3] est une vue schématique de différents modes de réalisation d'éléments filtrants.

L'unité de méthanisation selon l'invention a été conçue pour la méthanisation par voie sèche discontinue.

L'unité de méthanisation comporte un espace de chargement 1 destiné à accueillir une biomasse 2. La biomasse 2 peut comprendre des déchets verts telle que des plantes vertes, des branches, du fumier, du lisier, ou encore toute biomasse habituellement utilisée pour produire du biogaz.

L'unité de méthanisation comporte un moyen de percolation 3, permettant de répandre un liquide de percolation 4, par exemple du lisier, sur le dessus de la biomasse 2. Ce liquide de percolation 4 traverse la biomasse, et peut se charger de diverses particules à cette occasion. On appellera percolat 5, le liquide chargé obtenu.

L'unité de méthanisation comporte également un moyen d'évacuation 6 du percolat 5.

Selon l'invention, l'unité de méthanisation comporte un premier filtre 7, disposé de sorte à séparer l'espace de chargement 1 d'un espace de circulation 8, l'espace de circulation 8 étant situé entre le premier filtre 7 et le moyen d'évacuation 6. On permet ainsi au percolat 5 de circuler librement une fois qu'il est sorti de la biomasse 2, et jusqu'au moyen d'évacuation 6. On évite aussi de boucher le moyen d'évacuation 6 par de la biomasse. On permet aussi au percolat 5 sortant de la biomasse 2 de s'étaler dans l'espace de circulation 8, et de ne pas s'accumuler dans un endroit éloigné du moyen d'évacuation 6. Une telle accumulation ralentirait l'évacuation et augmenterait le risque d'une pression importante sur une des parois de l'unité de méthanisation.

Par exemple, le moyen d'évacuation 6 peut consister en une ouverture de la section d'un tuyau, disposé dans un coin de l'unité de méthanisation. Le premier filtre 7 peut présenter la forme d'une calotte sphérique, disposé autour de ce coin. On crée ainsi une distance entre la biomasse 2 et le moyen d'évacuation 6, qui permet au percolat 5 de circuler librement.

L'unité de méthanisation comporte un deuxième filtre 9, disposé entre l'espace de circulation et le moyen d'évacuation 6. Ce deuxième filtre 9 peut être disposé directement sur le moyen d'évacuation 6. Mais il peut aussi être disposé de sorte à ménager un deuxième espace de circulation 10, permettant au percolat 5 de circuler librement entre la sortie du deuxième filtre 9 et le moyen d'évacuation 6. Une telle configuration permet de disposer un deuxième filtre 9 d'une section bien plus grande que la section du moyen d'évacuation 6, et ainsi améliorer la vitesse de filtration, et/ou d'augmenter la durée d'utilisation du deuxième filtre 9 entre deux nettoyages.

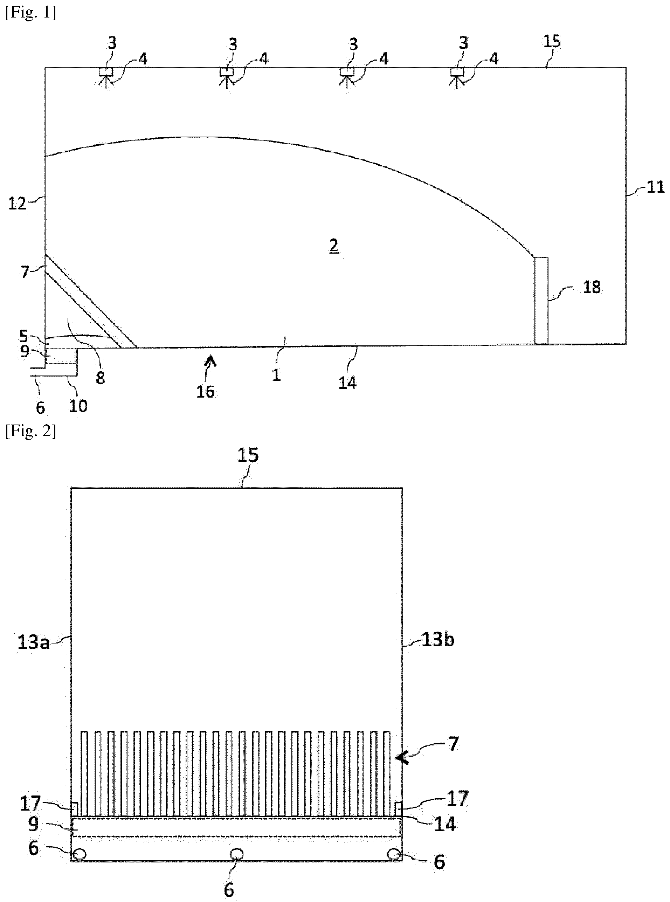

Selon un mode préféré de réalisation de l'invention représenté aux [Fig.1] et 2, l'unité de méthanisation est de forme parallélépipédique, de préférence rectangle, et comporte une paroi avant 11, une paroi arrière 12, deux parois latérales 13a, 13b, un sol 14 et un plafond 15.

Dans la suite de la description, le terme « avant » désigne la direction vers la paroi avant 11 de l'unité de méthanisation. De même, le terme « arrière » désigne la direction vers la paroi arrière 4 de l'unité de méthanisation.

Le sol 14 comporte de préférence une pente 16 descendant en direction de la paroi arrière 12. La pente 16 peut s'étendre sur l'intégralité du sol 14, ou sur une partie seulement du sol 14. La pente 16 permet de diriger le percolat 5 vers un moyen d'évacuation 6 du percolat hors de l'unité de méthanisation.

Le premier filtre 7 est disposé de telle sorte qu'il se trouve entre la biomasse 2, disposée dans l'espace de chargement 1, et l'espace de circulation 8. Selon un mode préféré de réalisation de l'invention, le premier filtre 7 peut par exemple s'étendre sur le sol 14 d'une paroi latérale 13a à l'autre 13b.

Le deuxième filtre 9 est disposé de telle sorte qu'il se trouve entre l'espace de circulation 8 et le moyen d'évacuation 6. Le deuxième filtre 9 peut par exemple s'étendre sur le sol 14, parallèlement et à l'arrière du premier filtre 7.

Il est entendu que pour le premier filtre 7 aussi bien que pour le deuxième filtre 9, le terme « sur le sol » ne se limite pas à un filtre intégralement disposé sur le sol, et qu'un filtre dont une partie serait disposée sous le plan formé par le sol 14, est couvert par cette expression, tant que le filtre est apte à filtrer le percolat 5 s'écoulant sur le sol. De plus le sol 14 peut être entièrement planaire, mais il peut aussi comporter des renfoncements tels que des rainures. Un filtre 7, 9 placé dans une rainure du sol est considéré dans la présente invention comme un filtre placé sur le sol.

Le deuxième filtre 9 est plus fin que le premier filtre 7, c'est-à-dire que le deuxième filtre 9 est tel qu'il ne laisse pas passer certains éléments que le premier filtre 7 laisse passer.

Le premier filtre 7 est par exemple configuré pour ne laisser passer que le liquide et les éléments dont au moins une dimension est inférieure à une dimension grossière, ladite dimension grossière étant comprise entre 10 et 80 mm, selon les produits constituant la biomasse 2. Dans un premier mode de réalisation du premier filtre 7, celui-ci comprend une pluralité d'éléments allongés, par exemple en plastique, en bois ou en métal, disposés parallèlement entre eux, s'appuyant d'un côté sur le sol 14 et de l'autre côté sur la paroi arrière 12 de l'espace de chargement 1. Les éléments allongés peuvent ainsi être disposés avec un angle compris entre 30 et 60°, typiquement un angle de 45° par rapport à l'horizontale. Un autre mode de réalisation possible est une grille métallique, par exemple verticale.

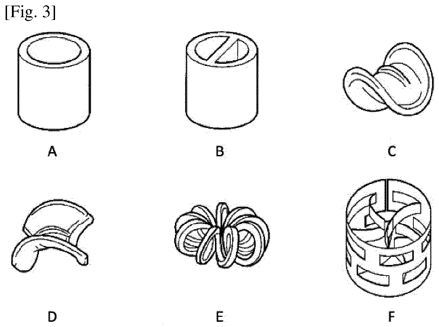

Le deuxième filtre 9 est par exemple configuré pour ne laisser passer que le liquide et les éléments dont au moins deux dimensions sont inférieures à une dimension fine, la dimension fine étant comprise entre 1 et 10 mm. Dans un premier mode de réalisation du deuxième filtre 9, celui-ci peut être réalisé par une enveloppe grillagée, par exemple faite d'une tôle percée de trous de 5 à 10 mm de diamètre, dans laquelle sont placés des éléments filtrants tels que des cailloux ou des éléments en plastique. Les éléments en plastique peuvent être des billes, des anneaux, ou encore des tubes de différentes formes, et de dimension variant de typiquement de 25 à 50 mm. Différents types de garnissage en vrac peuvent convenir, dont quelques exemples sont représentés en [Fig.3]. Il est notamment possible d'utiliser différents types d'anneaux tels que les anneaux de Raschig (exemple : élément A), les « Lessing rings » (exemple : élément B), les « Tellerette rings » (exemple : élément E) ou encore les « Pall rings » (exemple : élément F), et différentes types de selles telles que les « Berl saddle » (exemple : élément C) et les « Intalox saddle » (exemple : élément D).

Le premier filtre 7 et le deuxième filtre 9 sont placés de préférence de sorte à ménager un espace de circulation 8 entre les deux. Ainsi, le percolat 5 en sortie du premier filtre 7 peut librement s'étendre dans l'espace de circulation 8, et ainsi ne pas s'accumuler devant une partie seulement du deuxième filtre 9. Cette disposition permet d'optimiser l'utilisation de toute la capacité du deuxième filtre 9, et permet ainsi d'accélérer la filtration du percolat 5 par le deuxième filtre 9. L'espace d'écoulement peut par exemple s'étendre au sol sur une distance supérieure à 10 cm.

Le deuxième filtre 9 peut être disposé de sorte à ménager un deuxième espace de circulation 10 entre ledit deuxième filtre 9 et le moyen d'évacuation 6.

Par exemple le moyen d'évacuation 6 peut consister en une ou plusieurs ouvertures disposées dans l'arête entre la paroi arrière et le sol, de la section d'un tuyau, par exemple de diamètre 10 cm. Le deuxième filtre 9 peut être disposé sur toute la largeur de l'unité de méthanisation, dans cette même arête, d'une paroi latérale 13a à l'autre 13b.

Selon l'invention on ménage un deuxième espace de circulation 10 entre le deuxième filtre 9 et le moyen d'évacuation 6, par exemple par une rigole, de hauteur par exemple 5 à 10 cm, de largeur légèrement inférieure à la largeur du deuxième filtre 9, juste pour maintenir un support au niveau du sol pour ledit deuxième filtre, ou encore de même largeur que le deuxième filtre 9, et ce dernier s'insère partiellement dans la rigole, en laissant un espace avec le fond de la rigole. De cette façon on obtient un deuxième espace de circulation 10, tout le long de l'arête, et le percolat peut traverser le deuxième filtre 9 verticalement, sortir du deuxième filtre en coulant vers le bas, puis circuler librement vers l'une des ouvertures du moyen d'évacuation 6.

Selon un mode préféré de réalisation de l'invention, on dispose au moins une rigole longitudinale 17, reliant l'avant à l'arrière, sur sensiblement toute la longueur de l'unité de méthanisation. Cela permet à du percolat 5 écoulé vers l'avant de l'unité de méthanisation d'emprunter ladite rigole longitudinale 17 pour circuler jusqu'à l'arrière, et rejoindre le moyen d'évacuation 6. On évite ainsi une accumulation de percolat 5 à l'avant de l'unité de méthanisation, et une pression trop importante sur sa paroi avant 11. La rigole 17 peut consister en une conduite ouverte à l'avant et à l'arrière, par laquelle le percolat 5 peut circuler. On peut placer un filtre à l'entrée avant de la conduite, pour éviter un bouchage de l'entrée de la conduite par la biomasse 2. On peut par exemple disposer deux rigoles 17, une le long de chaque paroi latérale 13a et 13b.

La rigole longitudinale 17 peut aussi consister en une conduite perforée, les perforations permettant au percolat 5 d'entrer dans la conduite sur toute la longueur de l'espace de chargement 1, sans que la biomasse y entre. On peut réduire le risque de bouchage des perforations en plaçant un filtre entre la biomasse 2 et la rigole 17 sur toute sa longueur. La conduite perforée 17 peut également être ouverte à l'avant pour récupérer le surplus de percolat 5 s'étant écoulé vers l'avant.

En complément, on peut ajouter un mur 18 de protection de la paroi avant, dont la fonction est d'empêcher la biomasse de venir s'appuyer sur la paroi avant 11. Le mur peut être perméable au percolat ou on peut y disposer des trous, pour laisser passer le percolat 5, qui peut ensuite s'écouler par la ou les rigoles longitudinales 17. Le mur 18 permet aussi d'éviter que la biomasse 2 et le percolat ne s'appuient sur la paroi avant de l'unité de méthanisation et n'y génèrent une trop forte pression.

On procède à la percolation d'une unité de méthanisation par voie sèche discontinue en mettant successivement en oeuvre les étapes suivantes :
- versement d'un liquide de percolation sur une biomasse, ledit liquide de percolation devenant un percolat 5 en traversant la biomasse 2,
- filtration du percolat 5 par un premier filtre 7,
- filtration du percolat 5 par un deuxième filtre 9 plus fin que le premier filtre 7,
- évacuation du percolat 5 hors de l'unité de méthanisation par un moyen d'évacuation 6.

Selon un mode préféré de réalisation de l'invention, on dispose un espace de circulation 10 entre ledit premier filtre 7 et ledit moyen d'évacuation 6. Ceci permet au 5 de circuler librement vers toute la surface d'entrée du deuxième filtre 9, et améliore ainsi l'efficacité dudit deuxième filtre 9.

Selon un autre mode préféré de réalisation de l'invention, on dispose un deuxième espace de circulation 10 entre ledit deuxième filtre 9 et ledit moyen d'évacuation 6. Ceci permet au percolat 5 de circuler librement entre la sortie du deuxième filtre 9 et le moyen d'évacuation 6, et permet ainsi au percolat 5 de traverser ledit deuxième filtre 9 selon un parcours de traversée le plus court, en n'obligeant pas le percolat 5 à parcourir un trajet longitudinal ou transversal à l'intérieur du deuxième filtre 9.

## Revendications

1. Unité de méthanisation par voie sèche discontinue comportant un espace de chargement (1) destiné à accueillir une biomasse (2) à méthaniser, l'unité de méthanisation comprenant un moyen de percolation (3), et un moyen d'évacuation (6) d'un percolat (5), **caractérisée en ce que**
- l'unité de méthanisation comporte au moins un premier filtre (7) disposé de sorte à séparer l'espace de chargement (1) d'un espace de circulation (8),
- l'espace de circulation (8) étant situé entre le premier filtre (7) et le moyen d'évacuation (6),
- un deuxième filtre (9) étant disposé entre l'espace de circulation (8) et le moyen d'évacuation (6), ledit deuxième filtre (9) étant configuré plus fin que le premier filtre (7), de sorte à ne pas laisser passer certains éléments que le premier filtre (7) laisse passer.
- ledit deuxième filtre (9) étant disposé de sorte à ménager un deuxième espace de circulation (10) entre ledit deuxième filtre (9) et ledit moyen d'évacuation (6).

2. Unité de méthanisation selon la revendication précédente dans laquelle le premier filtre (7) ne laisse passer que le liquide et les éléments dont au moins une dimension est inférieure à une dimension grossière, ladite dimension grossière étant comprise entre 10 et 80 mm.

3. Unité de méthanisation selon l'une des revendications précédentes dans laquelle le deuxième filtre (9) ne laisse passer que le liquide et les éléments dont au moins deux dimensions sont inférieures à une dimension fine, la dimension fine étant comprise entre 1 et 10 mm.

4. Unité de méthanisation selon l'une des revendications précédentes, dans laquelle le deuxième filtre (9) comporte une cage grillagée dans laquelle sont placés des éléments filtrants tels que des cailloux ou des billes de plastique.

5. Unité de méthanisation selon l'une des revendications précédentes, comprenant un sol (14), une paroi avant (11), une paroi arrière (12), et deux parois latérales (13a, 13b), le moyen d'évacuation (6) étant disposé au niveau de l'intersection entre le sol (14) et la paroi arrière (12), et le premier filtre (7) s'étendant selon un plan incliné entre le sol (14) et la paroi arrière (12), d'une paroi latérale à l'autre (13a, 13b).

6. Unité de méthanisation selon la revendication précédente, dans laquelle le premier filtre (7) comporte des éléments allongés, par exemple en bois, disposés parallèlement entre eux, s'appuyant d'un côté sur le sol (14) et de l'autre côté sur la paroi arrière (12) de l'unité de méthanisation.

7. Unité de méthanisation selon l'une des revendications 5 ou 6, comportant au moins un moyen d'écoulement couvert (17) disposé au sol (14) le long d'une paroi latérale (13a, 13b) de l'unité de méthanisation, permettant de faire couler du percolat (5) de la paroi avant (11) vers la paroi arrière (12) sans traverser la biomasse (2).

8. Procédé de percolation d'une unité de méthanisation par voie sèche discontinue comportant successivement les étapes suivantes :
- versement d'un liquide de percolation (4) sur une biomasse (2), ledit liquide de percolation (4) devenant un percolat (5) en traversant la biomasse (2),
- filtration du percolat (5) par un premier filtre (7),
- circulation du percolat (5) dans un espace de circulation (8),
- filtration du percolat (5) par un deuxième filtre (9) plus fin que le premier filtre (7),
- circulation du percolat (5) dans un deuxième espace de circulation (10),
- évacuation du percolat (5) hors de l'unité de méthanisation.

## Patentansprüche

1. Diskontinuierliche Trockenmethanisierungseinheit mit einem Beschickungsraum (1) zur Aufnahme einer zu methanisierenden Biomasse (2), wobei die Methanisierungseinheit ein Perkolationsmittel (3) und ein Abführmittel (6) für ein Perkolat (5) umfasst, **dadurch gekennzeichnet, dass**
- die Methanisierungseinheit mindestens einen ersten Filter (7) umfasst, der so angeordnet ist, dass er den Beschickungsraum (1) von einem Zirkulationsraum (8) trennt,
- wobei der Zirkulationsraum (8) zwischen dem ersten Filter (7) und dem Abführmittel (6) angeordnet ist,
- ein zweiter Filter (9) zwischen dem Zirkulationsraum (8) und dem Abführmittel (6) angeordnet ist, wobei der zweite Filter (9) feiner als der erste Filter (7) konfiguriert ist, so dass er bestimmte Elemente, die der erste Filter (7) durchlässt, nicht durchlässt.
- wobei der zweite Filter (9) so angeordnet ist, dass ein zweiter Zirkulationsraum (10) zwischen dem zweiten Filter (9) und dem Abführmittel (6) entsteht.

2. Methanisierungseinheit nach dem vorhergehenden Anspruch, bei der der erste Filter (7) nur Flüssigkeit und Elemente durchlässt, von denen mindestens eine Abmessung kleiner als eine grobe Abmessung ist, wobei die grobe Abmessung zwischen 10 und 80 mm liegt.

3. Methanisierungseinheit nach einem der vorhergehenden Ansprüche, bei der der zweite Filter (9) nur Flüssigkeit und Elemente durchlässt, bei denen mindestens zwei Abmessungen kleiner als eine Feinabmessung sind, wobei die Feinabmessung zwischen 1 und 10 mm liegt.

4. Methanisierungseinheit nach einem der vorhergehenden Ansprüche, wobei der zweite Filter (9) einen Drahtkäfig umfasst, in dem Filterelemente wie Kieselsteine oder Kunststoffkugeln angeordnet sind.

5. Methanisierungseinheit nach einem der vorhergehenden Ansprüche, umfassend einen Boden (14), eine Vorderwand (11), eine Rückwand (12) und zwei Seitenwände (13a, 13b), wobei das Abzugsmittel (6) am Schnittpunkt zwischen dem Boden (14) und der Rückwand (12) angeordnet ist und sich der erste Filter (7) entlang einer geneigten Ebene zwischen dem Boden (14) und der Rückwand (12) von einer Seitenwand zur anderen (13a, 13b) erstreckt.

6. Methanisierungseinheit nach dem vorhergehenden Anspruch, wobei der erste Filter (7) längliche Elemente, z. B. aus Holz, umfasst, die parallel zueinander angeordnet sind und sich auf der einen Seite auf dem Boden (14) und auf der anderen Seite auf der Rückwand (12) der Methanisierungseinheit abstützen.

7. Methanisierungseinheit nach einem der Ansprüche 5 oder 6, mit mindestens einem am Boden (14) entlang einer Seitenwand (13a, 13b) der Methanisierungseinheit angeordneten, abgedeckten Fließmittel (17), das es ermöglicht, Perkolat (5) von der Vorderwand (11) zur Rückwand (12) fließen zu lassen, ohne die Biomasse (2) zu durchströmen.

8. Verfahren zur Perkolation einer diskontinuierlichen Trockenmethanisierungseinheit, das nacheinander die folgenden Schritte umfasst:
- Gießen einer Perkolationsflüssigkeit (4) auf eine Biomasse (2), wobei die Perkolationsflüssigkeit (4) beim Durchlaufen der Biomasse (2) zu einem Perkolat (5) wird,
- Filtern des Perkolats (5) durch einen ersten Filter (7),
- Zirkulation des Perkolats (5) in einem Zirkulationsraum (8),
- Filtern des Perkolats (5) durch einen zweiten Filter (9), der feiner ist als der erste Filter (7),
- Zirkulation des Perkolats (5) in einen zweiten Zirkulationsraum (10),
- Abfuhr des Perkolats (5) aus der Trockenmethanisierungseinheit.

## Claims

1. Batch dry methanisation unit comprising a loading space (1) intended to receive a biomass (2) to be methanised, the methanisation unit comprising a percolation means (3), and a discharge means (6) for discharging a leachate (5), **characterised in that**
- the methanisation unit comprises at least a first filter (7) arranged to separate the loading space (1) from a circulation space (8),
- the circulation space (8) being located between the first filter (7) and the discharge means (6),
- a second filter (9) being arranged between the circulation space (8) and the discharge means (6), said second filter (9) being configured finer than the first filter (7), so as not to let through certain elements that the first filter (7) lets through
- said second filter (9) being arranged so as to provide a second circulation space (10) between said second filter (9) and said discharge means (6).

2. Methanisation unit according to one of the previous claims wherein the first filter (7) only allows the passage of liquid and elements of which at least one dimension is smaller than a coarse dimension, said coarse dimension being comprised between 10 and 80 mm.

3. Methanisation unit according to one of the previous claims in which the second filter (9) only lets through the liquid and the elements of which at least two dimensions are smaller than a fine dimension, the fine dimension being between 1 and 10 mm.

4. Methanisation unit according to one of the previous claims, in which the second filter (9) comprises a wire mesh cage in which filtering elements such as pebbles or plastic balls are placed.

5. Methanisation unit according to one of the previous claims, comprising a floor (14), a front wall (11), a rear wall (12), and two side walls (13a, 13b), the discharge means (6) being disposed at the intersection between the floor (14) and the rear wall (12), and the first filter (7) extending in an inclined plane between the floor (14) and the rear wall (12), from one side wall (13a) to the other (13b).

6. Methanisation unit according to the previous claim, wherein the first filter (7) comprises elongated elements, for example made of wood, arranged parallel to each other, resting on one side on the floor (14) and on the other side on the rear wall (12) of the methanisation unit.

7. Methanisation unit according to one of claims 5 or 6, comprising at least one covered flow means (17) arranged on the floor (14) along a side wall (13a, 13b) of the methanisation unit, allowing leachate (5) to flow from the front wall (11) to the rear wall (12) without passing through the biomass (2).

8. Method for percolating a dry batch methanisation unit comprising the following steps in succession:
- pouring a percolation liquid (4) onto a biomass (2), said percolation liquid (4) becoming a leachate (5) as it passes through the biomass (2),
- filtration of the leachate (5) by a first filter (7),
- circulating of the leachate (5) in a circulation space (8),
- filtration of the leachate (5) by a second filter (9) which is finer than the first filter (7),
- circulating of the leachate (5) in a second circulation space (10),
- discharge of the leachate (5) from the methanisation unit.
